# EUROPEAN PATENT APPLICATION

(11) **EP 3 155 997 A1**
(43) Date of publication of application: **19.04.2017**
(21) Application number: 15003531.9
(22) Date of filing: 11.12.2015
(51) Int. Cl.: A61B 50/15, A61B 50/20, A61B 50/24, A61B 50/33

(54) **TABLE FOR HEMODYNAMIC PROCEDURES AND MODULAR SYSTEM COMPRISING THE TABLE**

(30) Priority: 15.10.2015 PL 41438515
(71) Applicant: Innovations for Heart and Vessels Sp. z o.o., 40-171 Katowice (PL)
(72) Inventor: Buszman, Piotr, 40-761 Katowice (PL); Buszman, Pawel, 40-748 Katowice (PL); Milewski, Krzysztof, 40-748 Katowice (PL)
(74) Representative: Gizinska-Schohe, Malgorzata

(57) **Abstract**

The invention refers to the table for hemodynamic procedures for attaching the elements of medical equipment, comprising the tabletop and supports, characterised in that the first (**2**) and second (**2'**) supports placed opposite each other have the inner edges, along with the bottom edges of the tabletop (**1**) of the table shaped as at least one ergonomically rounded recess (**5**, **6**), the tabletop of the table being provided from the top, at least in part of its operating surface, with a part (**4**) comprising the connecting elements (**11**) of the first type receiving the connecting elements (**12**) of the second type engaging them, placed on the elements of the attached medical equipment.

## Description

The object of the invention is a table for hemodynamic procedures, i.e. cardiological procedures, in particular conducted percutaneously, in which medical devices are introduced into the patient's blood vessels or into the heart percutaneously, e.g. via the radial or femoral artery. The table is useful for all kinds of percutaneous hemodynamic procedures, in which it is necessary to operate medical devices and to stabilise their position, or to promptly readjust them, or to add devices of differing functionality in progress of the conducted procedure. The object of the invention is also a modular system comprising the table for the procedures along with further attachable elements of the medical equipment.

In progress of the conducted hemodynamic procedures, typical medical equipment is used, which is not adjusted to the specifics of these procedures in any particular manner. Due to the increasing number of procedures involving the heart, e.g. the coronary angioplasty procedures, it is necessary to improve the working conditions and the efficiency of the surgeon and the assistants. Considering the complexity of the hemodynamic procedures, the medical personnel should have at their disposal the tools enabling precision, hygiene and safety of those procedures.

The purpose of the present invention is the development of a new construction of the table with a relatively simple construction, which enables its ergonomic adjustment and stabilisation using the shape of the human limbs as the substrate being the foundation for the table. The table also enables removable attachment of the medical equipment, precise control of its location or setting. The individual elements of the medical equipment as separate products may be arranged, sterilised, preserved etc. at will, regardless of the table itself.

The object of the invention is the table for hemodynamic procedures for attaching the elements of medical equipment, comprising the tabletop and the supports, characterised in that the first and second supports placed opposite each other have the inner edges, along with the bottom edges of the tabletop of the table shaped in the form of at least one ergonomically rounded recess, the tabletop of the table being provided from the top, at least in part of its operating surface, with a part comprising the connecting elements of the first type receiving the cooperating with them connecting elements of the second type, placed on the elements of the attached medical equipment.

The table comprises two rounded recesses - first and second, located next to it. The connecting elements of the first type have the form of convex protrusions, and the connecting elements of the second type have the form of concave recesses whose shape mirrors the abovementioned protrusions, or vice versa: the elements of the first type have the form of recesses, and the elements of the second type have the form of protrusions.

The object of the invention is also a modular system of the table comprising the tabletop and the supports, as well as the elements of medical equipment attached to the table characterised in that the table comprises the first and second supports placed opposite each other, which have the inner edges along with the bottom edges of the tabletop of the table, shaped in the form of at least one ergonomically rounded recesses, the tabletop of the table being provided from the top, at least in part of its operating surface, with a part comprising the connecting elements of the first type receiving the cooperating with them connecting elements of the second type, with which are provided on their base or body the attachable elements of the medical equipment constituting the further parts of the modular system.

The developed concept of the medical table for percutaneous procedures involving the heart ensures optimal adjustment of the table during the procedures, and due to the system of clips it will be possible to stably attach the elements of the equipment. The modular set ensures both easy disassembly of the system into components as well as efficient washing, cleaning and sterilisation. It is also possible to construct the system from suitable types of plastic as disposable equipment. A preferable effect will be the possibility to select the equipment at will, depending on the needs, i.e. along with one table only certain remaining elements of the set may be used up.

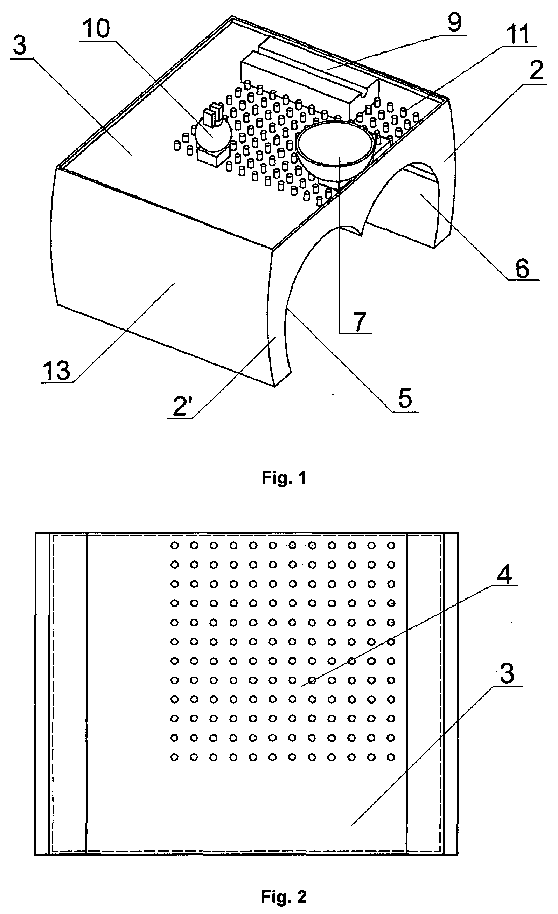

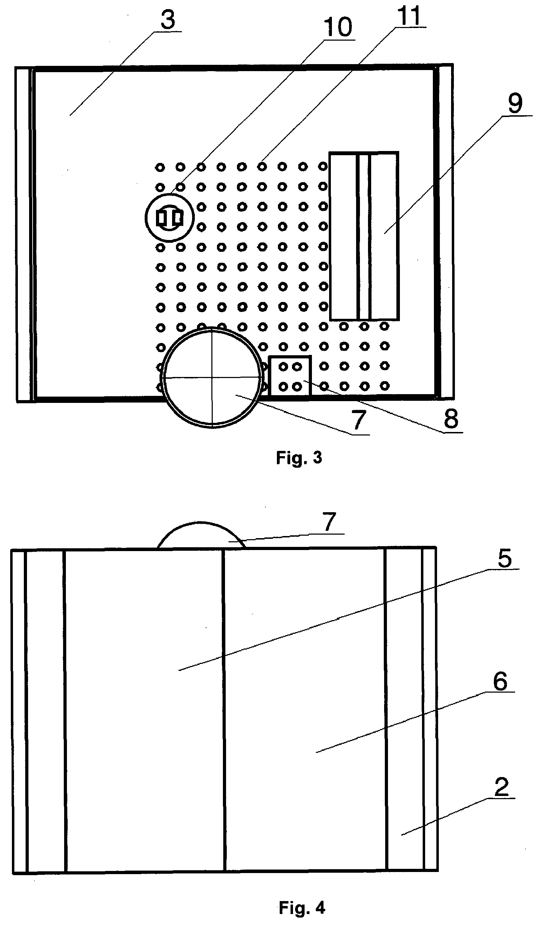

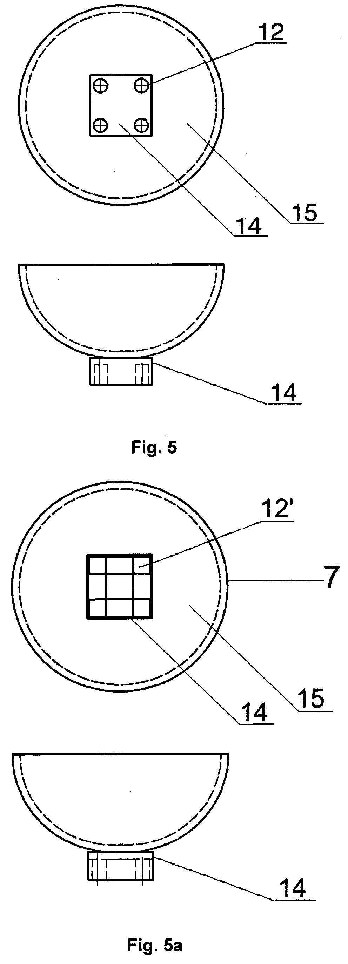

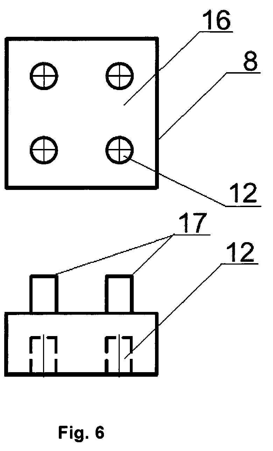

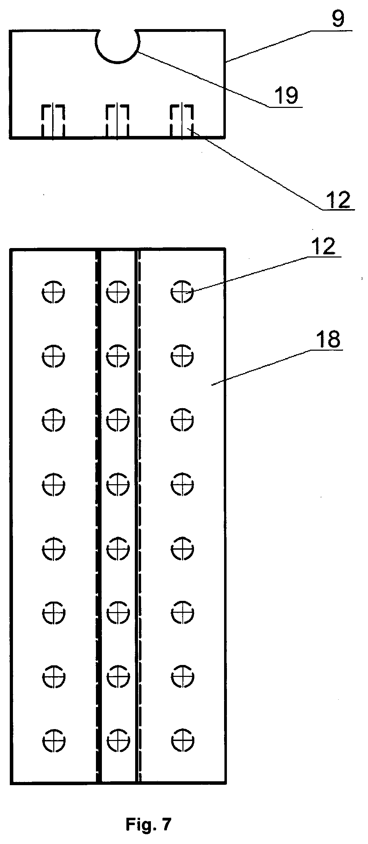

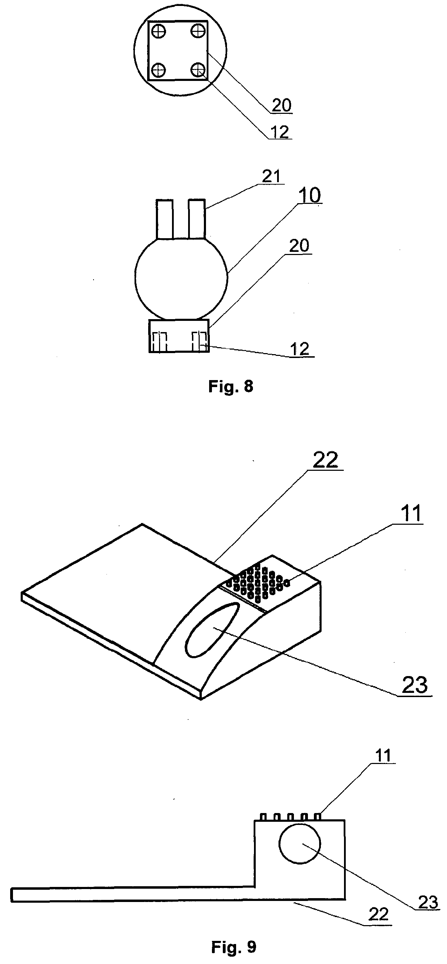

The object of the invention is presented in an embodiment in the drawing, in which fig. 1 presents a general view of the medical table along with the listed sample medical devices, fig. 2 shows the table in a top view without the medical devices, fig. 3 presents the table in a top view with the medical devices as presented in fig. 1, fig. 4 shows the table from fig. 1 in a bottom view, fig. 5 shows the bowl viewed from the bottom (upper drawing) and the side (lower drawing) in the first embodiment, in which the base of the bowl is provided with the connecting elements of the second type in the form of recesses with a cylindrical shape, fig. 5a shows the bowl viewed from the bottom (upper drawing) and the side (lower drawing) in the second embodiment, in which the base of the bowl is provided with the connecting elements of the second type in the form of recesses with a cuboidal shape, fig. 6 shows a handle of the catheter viewed from the bottom (upper drawing) and the side (lower drawing), fig. 7 shows the guide handle viewed from the bottom (upper drawing) and the side (lower drawing), fig. 8 shows the handle of the Y-connector viewed from the bottom (upper drawing) and the side (lower drawing), and fig. 9 shows a table cover for the radial artery procedure in a general (upper drawing) and side view (lower drawing) from the side of the wider end of the through opening.

The medical table in the embodiment is a one-piece solid comprising the tabletop 1 and two supports placed opposite each other - the first support 2 and the second support 2' projecting down from the bottom surface of the tabletop. The supports 2 and 2' are placed on the two edges of the tabletop, it being possible for them to be placed only along a part of the edge of the tabletop. It is preferable for the supports to be placed symmetrically on the two sides of the tabletop, since it benefits the stability of the whole table. The supports generally project down from the surface of the tabletop, being able to project down at an approximate angle of 90° relative to the surface of the tabletop. This may also be an angle falling within the range from 60 to 120°. The supports preferably have such height that they rest freely both on the patient's limbs as well as on the surfaces located below, e.g. the surface of the surgical bed, constituting a firm support. At least a fragment of the tabletop of the table constitutes the part 4 provided with the connecting elements, which ensures the creation of an area intended for attaching the medical devices which may be described as devices pressed inside. Preferably, the table also comprises the smooth part **3**, which lacks the connecting elements. On their inner side facing the opposite support, the supports **2, 2'** of the table, along with the lower edge of the tabletop, form at least one ergonomic rounded recess, it being preferable for it to be two recesses: first **5** and second 6 - placed next to each other. The ergonomic shape of the recess (recesses) is understood as adjusted to the typical size of human lower/upper limbs. These recesses should be ovoid, have gentle edges lacking any elements able to cause injury. The rounding of the recesses may be approximately cylindrical, adjusted to the shape of an arm or a thigh. A gentle indentation of the inner surface of the recesses **5** and **6** is also possible in order to strengthen the placing of the table on the patient's limbs. It is also possible for the table to be adjusted in such a manner that the supports **2**, **2'** are movable with respect to each other, in order to enable widening or narrowing of the space of the recesses **5** and **6**. The supports **2**, **2'** forming the recesses **5**, **6** may also be rounded on their outer side walls **13,** but such form is not absolutely necessary. It is possible to design the recesses in such a manner that their interior is rounded, but their walls **13** may be flat, one-plane.

Part **4** of the tabletop comprising the connecting elements **11** of the first type placed on the tabletop is meant for placing upon it such sample auxiliary medical devices as the bowl **7** for fluids, the handle **8** of the catheter for mounting intravascular catheters, the handle **9** of the guide or the handle 10 for the Y-connector. The abovementioned medical devices are provided with bases which, from the side abutting on the tabletop of the table, are provided with the connecting elements **12** of the second type. At the same time, on the side opposite to the base of the medical device is its functional tip useful in medical procedures. Therefore, the bowl **7** comprises the base **14** of the bowl and the dome **15,** the handle **8** of the catheter comprises the base **16** of the handle of the catheter and the arms **17,** the guide handle **9** comprises the base **18** of the guide handle and a sculpted groove **19** of the guide handle, while the handle **10** for the Y-connector comprises the base **20** of the handle of the Y-connector and the arms **21** of the handle, and the table cover for radial access comprises a stabilising base **22** and a through opening **23** for the arm.

The abovementioned medical devices are only examples, and for a person skilled in the art it is obvious that it is also possible to create other devices or their elements compatible with the whole system. It is also possible to create a universal connector provided with a base such as the ones mentioned above, but with the possibility of traditional (e.g. clipped or screwed) mounting of further traditional medical devices.

The arms comprised in the abovementioned devices and holding the medical devices may be designed in various sizes/types and may allow for the use with various medical devices. It is also obvious that the form/shape of the presented bowl is only demonstrative and may be modified.

In the abovementioned manner, a modular system is created for the table and the medical devices, whose mutual engagement (fitting) is ensured via a system of connecting elements of the first type and of the second type. These terms refer to the connecting elements grouped into pairs, meaning that each connecting element of the first type cooperates with a connecting element of the second type. All the elements of the first type have the same shape and size and they function based on the same principle. Also, the elements of the second type have the same shape and size. Connections are created in the form of pairs of "conforming" shape. Each pair of connecting elements ensures a detachable, mechanical, fitted connection of multiple use. A connection is formed based on the "plug" and "socket" principle, i.e. a shape-force fit. The connecting elements on the tabletop of the table are integrated with the elements located on the bases (bodies) of the attached medical devices.

For example, the shape of the connecting element of the first type mirrors the connecting element of the second type, e.g., as shown in fig. 1, the elements of the first type constitute cylindrical (with a circular cross-section) protrusions projecting from the surface of the tabletop of the table. Each connecting element has an identical shape. The connecting elements of the second type shown e.g. in fig. 5 are adjusted to them in terms of shape - these are concave (hollowed) recesses with a cylindrical shape, with the size corresponding to the cylindrical protrusions, which is the form of the connecting elements of the first type shown in fig. 1. Attachment of medical devices to the tabletop of the table is accomplished by pressing the devices with the recesses onto the tabletop comprising the protrusions. For efficient functioning of the connections, it is obvious that constant, predefined clearances must be maintained between the connecting elements.

The connection system of the described plug connections may also be realised in a modified manner. E.g., fig. 5a shows the connecting elements of the second type in the form of cuboidal recesses whose size is adjusted to the size of the elements of the first type in the form of cylindrical protrusions. In such type the obtained connection is created by pressing the cuboidal recesses onto the projecting cylindrical protrusions.

It is possible to envisage numerous other forms of the engaging connecting elements grouped into pairs. These may be convex elements shaped as cuboids pressed into the cylindrically shaped recesses etc.

It is also possible to develop a different type of connecting elements in the conforming pairs - these may be connections with latches, springs, hooks, tongue and groove connections etc. The connecting elements may comprise indentations, springing protrusions, bulges adjusted to the recesses etc.

It is also possible to realise the invention in such a manner that the device bases of various sizes are provided, with the shape of the connecting elements adjusted to the connecting elements on the tabletop of the table, and subsequently the medical devices are mounted onto that special base provided with specific (compatible with the system) connecting elements in an arbitrary manner, by gluing, welding or riveting.

References in the drawings:
- 1.: table
- 2.: first support
- 2'.: second support
- 3.: smooth part of the tabletop of the table
- 4.: part of the tabletop comprising the connecting elements
- 5.: first recess of the table
- 6.: second recess of the table
- 7.: bowl
- 8.: handle of the catheter
- 9.: guide handle
- 10.: handle of the Y-connector
- 11.: connecting element of the first type
- 12.: connecting element of the second type
- 13.: side wall of the support
- 14.: base of the bowl
- 15.: dome of the bowl
- 16.: base of the handle of the catheter
- 17.: arms of the handle of the catheter
- 18.: base of the guide handle
- 19.: sculpted groove of the guide handle
- 20.: base of the handle of the Y-connector
- 21.: arms of the handle of the Y-connector
- 22.: stabilising base
- 23.: through opening for the arm

## Claims

1. The table for hemodynamic procedures for attaching the elements of medical equipment, comprising the tabletop and supports, **characterised in that** the first (**2**) and second (**2'**) supports placed opposite each other have the inner edges, along with the bottom edges of the tabletop (**1**) of the table shaped in the form of at least one ergonomically rounded recess (**5, 6**), the tabletop of the table being provided from the top, at least in part of its operating surface, with a part (**4**) comprising the connecting elements (**11**) of the first type receiving the cooperating with them connecting elements (**12**) of the second type, placed on the elements of the attached medical equipment.

2. The table according to claim 1, **characterised in that** it comprises two rounded recesses - first (**5**) and second (**6**), located next to it.

3. The table according to claim 1, **characterised in that** the connecting elements (**11**) of the first type have the form of convex protrusions, and the connecting elements (**12**) of the second type have the form of concave recesses whose shape mirrors the abovementioned protrusions, or vice versa: the elements (**11**) of the first type have the form of recesses, and the elements (**12**) of the second type have the form of protrusions.

4. The modular system of the table comprising the tabletop and the supports as well as the elements of the medical equipment attached to the table, **characterised in that** the table comprises the first (**2**) and second (**2'**) supports placed opposite each other, which have the inner edges along with the bottom edges of the tabletop (**1**) of the table, shaped in the form of at least one ergonomically rounded recess (**5, 6**), the tabletop of the table being provided from the top at least in part of its operating surface with the part (**4**) comprising the connecting elements (**11**) of the first type receiving the cooperating with them connecting elements (**12**) of the second type, with which are provided, on their base or body, the attachable elements of the medical equipment constituting the further parts of the modular system.
